# EUROPEAN PATENT SPECIFICATION

(11) **EP 1 385 497 B1**
(45) Date of publication and mention of the grant of the patent: **08.03.2006**
(21) Application number: 02764251.1
(22) Date of filing: 19.04.2002
(51) Int. Cl.: A61K 31/198, A61P 35/00, A61P 35/04

(54) **PREVENTION OR TREATMENT OF BREAST, PROSTATE AND/OR CERVICAL CANCER WITH N,N-DIMETHYLGLYCINE**
PRÄVENTION ODER BEHANDLUNG VON BRUST-, PROSTATA- UND/ODER GEBÄRMUTTERHALSKREBS MIT N,N-DIMETHYLGLYCIN
PREVENTION OU TRAITEMENT DU CANCER DU SEIN, DE LA PROSTATE ET/OU DU COL DE L'UTERUS A L'AIDE DE N,N-DIMETHYLGLYCINE

(30) Priority: 20.04.2001 US 838571
(43) Date of publication of application: 04.02.2004
(73) Proprietor: Foodscience Corporation, Essex Junction, VT 05453 (US)
(72) Inventor: KENDALL, Roger, V., Westford, VT 05494 (US)
(74) Representative: Ziebig, Marlene
(86) International application number: PCT/US2002/012425
(87) International publication number: WO 2002/085345

(56) References cited:
- US-A- 4 385 068
- US-A- 4 994 492
- US-A- 5 026 728
- REAP E A ET AL: "IMMUNOMODULATING AND ANTI-TUMOR ACTIVITIES OF THE INTERMEDIARY METABOLITE DIMETHYLGLYCINE DMG" FASEB JOURNAL, vol. 4, no. 7, 1990, page A1767, XP009046507 & JOINT MEETING OF THE AMERICAN SOCIETY FOR BIOCHEMISTRY AND MOLECULAR BIOLOGY AND THE AMERICAN ASSOCI ISSN: 0892-6638

## Description

This invention relates to the use of N,N-dimethylglycine (DMG) to prevent or treat breast, prostate and/or cervical cancer in man or other animals.

Dimethylglycine is an intermediary metabolite and amino acid found in low levels in many foods, and is produced in the body from choline. DMG is an endogenous compound and an enzyme system in the body effectively converts the substance into metabolites that are either used by the body or are safely excreted from the body.

A great deal of research has been carried out in recent years on the physiological effects and potential health benefits of N,N-dimethylglycine.

Referring to previous work, U.S. Pat. No. 4,385,068, discloses treating irradiated animals with a derivative of this compound to alleviate the effects of excess radiation on the immune system.
U.S. Pat. No. 4,994,492, discloses treating melanoma tumor by administering N,N-dimethylglycine or a pharmaceutically acceptable salt thereof.

The literature is replete with articles using the term vitamin "B-15". The term "vitamin-B-15" is often inaccurately referred to as NN-dimethylglycine; however the term "B-15" is imprecise and different brands have completely different compositions. A great deal of research has been carried out, particularly in the Soviet Union, on a substance which the Russian researchers call calcium pangamate (the calcium salt of pangamic acid). Calcium pangamate has also been termed "vitamin B-15".

### Summary of the Invention

It has now been found that DMG is effective for the treatment, inhibition and prevention of cervical, breast and prostate cancer.

In one aspect, this invention relates to inhibiting the metastasis of cervical, breast and prostate cancers by administering to a patient with cervical, breast and/or prostate cancer a metastasis-inhibiting amount of N,N-dimethylglycine or a pharmaceutically acceptable salt thereof.

In another aspect this invention relates to inhibiting the formation of cervical, breast or prostate cancer cells in a patient or for decreasing the risk of cervical, breast or prostate cancer in a patient by administering N,N-dimethylglycine or a pharmaceutically acceptable salt thereof to a patient who is at risk for cervical, breast or prostate cancer.

In another aspect this invention relates the treatment of cervical, breast or prostate cancer comprising administering N,N-dimethylgtycine or a pharmaceutically acceptable salt thereof to a patient with a cervical, breast or prostate cancer.

N,N-Dimethylglycine is a compound of the formula:

(CH₃)₂ NCH₂ COOH

DMG or a pharmacologically acceptable salt thereof, can be used to treat, prevent or inhibit cervical, breast and/or prostate cancer. DMG or a pharmaceutically acceptable salt thereof, can also help prevent, treat and contain metastasis of cervical, breast and/or prostate cancer. The applicability of DMG in cancer immunotherapy is demonstrated in both in-vitro cell culture experimental methods an in vivo animal experiments in which DMG has been shown to have a positive effect against the spread and proliferation of cancer cells.

In industrialized countries the second cause of death after heart disease is cancer. Breast, cervical and prostate cancer predominate in many countries. Cancer is the result of an uncontrolled local proliferation of cells with invasion of adjacent normal structures. Metastasis occurs when the cancer spreads via bloodstream or lymph nodes or within a body cavity. Breast cancer is the most common type of cancer among women in the United States. There are different types of breast cancers, but the most common type, ductal carcinoma, begins in the lining of the breast's ducts.

Another type, lobular carcinoma arises in the lobules. Sometimes breast cancer can spread to other parts of the body such as the lymph nodes, most commonly those under the arm. If and when it invades other parts of the body it is called "metastatic disease" or "distant disease". Most breast patients are women but male breast cancer also occurs, i.e., with 1% the frequency of female breast cancer. Domestic mammals such as dogs, horses, etc. are equally susceptible to mammary cancer.

Human breast cancer, also known as mammary cancer (and interchangeably used in the text herewith), is a disease which can result from several factors such as ionizing radiation, diet, familial history or exposure to genetic mutagens.

Prostate cancer is the most common malignancy affecting adult males in the population. It is diagnosed in over 150,000 males in the U.S. annually. The prostate is a 20-gram gland, which is located at the base of the bladder and surrounds the urethra and consists of five lobes; the anterior, posterior, median and right and left lateral lobes. Since the prostate is a gland, the most common type of cancer is called adenocarcinoma.

Each year, about 15,000 women in the United States are diagnosed with cancer of the cervix, also called cervical cancer. Like most cancers, it is named for the part of the body in which it begins. Cancers of the cervix also are named for the type of cell in which they begin. Most cervical cancers are squamous cell carcinomas. Squamous cells are thin, flat cells that form the surface of the cervix.

When cancer spreads to another part of the body, the new tumor has the same kind of abnormal cells and the same name as the original (primary) cancer. For example, if cervical cancer spreads to the bones, the cancer cells in the bones are cervical cancer cells. The disease is called metastatic cervical cancer.

The treatment of the instant invention involves the administration of DMG or a pharmaceutically acceptable salt thereof to a subject, including but not limited to mammals, including humans. Suitable hosts for the prevention or inhibition aspects of the invention include those with genetic disposition to, family history and or exposure to human cancer causing agents (e.g., radiation, mutagens etc) of , the cancer of concern, prostate, breast and/ or cervical. Inhibition of mutagenesis can, of course also be achieved in hosts already having such a cancer.

One aspect of this invention involves administering to a patient, e.g. a human who has been diagnosed as having cervical, breast and/or prostate cancer. It is preferred that the tumor has not yet metastasized. DMG should be administered as soon as possible even though the cancer or suspected cancer has not been fully characterized. Clinical experience indicates that DMG will not interfere with most other drug therapies generally, and can be given to patients regardless of age, sex, cancer type or general health status either by oral or IV routes. DMG can also be expected to improve the immune status of cancer patients with cervical, breast and prostate cancer patients within 10 days. Positive evidence of effective treatment against cervical, breast and/or prostate cancer should be evident in as early as 14-28 days.

The DMG used in the instant invention can be processed in accordance with conventional methods of galenic pharmacy to produce medicinal agents for administration to patients, e.g. mammals, including humans and, e.g., the other animals mentioned herein.

The DMG used in this invention can be employed in admixture with conventional excipients, i.e. pharmaceutically acceptable organic or inorganic carrier substances suitable for parenteral, enteral (e.g., oral) application which do not deleteriously react with the active compound. Suitable pharmaceutically acceptable carriers include but are not limited to water, salt solutions, alcohols, gum arabic, vegetable oils, benzyl alcohols, polyethylene glycols, gelatine, carbohydrates such as lactose, amylose or starch, magnesium stearate, talc, silicic acid, viscous paraffin, perfume oil, fatty acid monoglycerides and diglycerides, pentaerythritol fatty acid esters, hydroxy methylcellulose, polyvinyl pyrrolidone, etc. The pharmaceutical preparations can be sterilized and if desired mixed with auxiliary agents, e.g. lubricants, preservatives, stabilizers, wetting agents, emulsifiers, salts for influencing osmotic pressure, buffers, coloring, flavoring and/or aromatic substances and the like which do not deleteriously react with the active compound. They can also be combined where desired with other active agents.

For parenteral application, particularly suitable are injectable, sterile solutions, preferably oily or aqueous solutions, as well as suspensions, emulsions, or implants. Ampules are convenient unit dosages.

For enteral application, particularly suitable are tablets, dragees, liquids, drops, or capsules.

The present invention also relates to useful forms of N, N-Dimethylglycine as disclosed herein, such as pharmaceutically acceptable salts and prodrugs of N,N-Dimethylglycine. Pharmaceutically acceptable salts include those in which the main compound functions as a base, e.g., hydrochloride, methane sulfonate, camphor sulfonate, as well as those for which the main compound functions as an acid, e.g., sodium, chloride salts etc.

Generally, when used in the treatment of or inhibition of metastasis of cervical, breast and/or prostate cancer, the compounds of this invention are dispensed in unit dosage form comprising 1-500 mg/kg/day, preferably 20-200 mg/kg/day, and most preferably 50-100 mg/kg/day in a pharmaceutically acceptable carrier. The daily dosage of the compounds according to this invention, when used to prevent metastasis or prevent the formation of cervical, breast and prostate cancers is generally about 1-500 mg/kg/day, preferably 10-100 mg/kg/day and most preferably 20-50 mg/kg/day. Generally, treatment or inhibition of metastasis dosage levels are higher than prevention dosage levels. Once cervical, breast and/or prostate cancer remission is diagnosed the dosage levels can be reduced to preventative dosage levels. When administered orally, the dosage can be in a single or divided dosages every 2-24 hours, preferably every 4 hours; when administered intraperitoneally or intramuscularly initially it should be administered daily, and thereafter periodically, preferably at least every third day.

As mentioned, DMG can be administered concurrently or alternately with other therapeutic treatments conventionally employed in cancer therapy, e.g. irradiation, surgery, chemotherapeutic agents, and other acceptable therapies designed to reduce the tumor load.

It will be appreciated that the actual preferred amounts of active compound in a specific case will vary according to the particular compositions formulated, the mode of application, and the particular situs and organism being treated. Dosages for a given host can be determined using conventional considerations, e.g., by customary comparison of the differential activities of the subject compounds and of a known agent, e.g., by means of an appropriate, conventional pharmacological protocol.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

### Brief Description of the Drawings

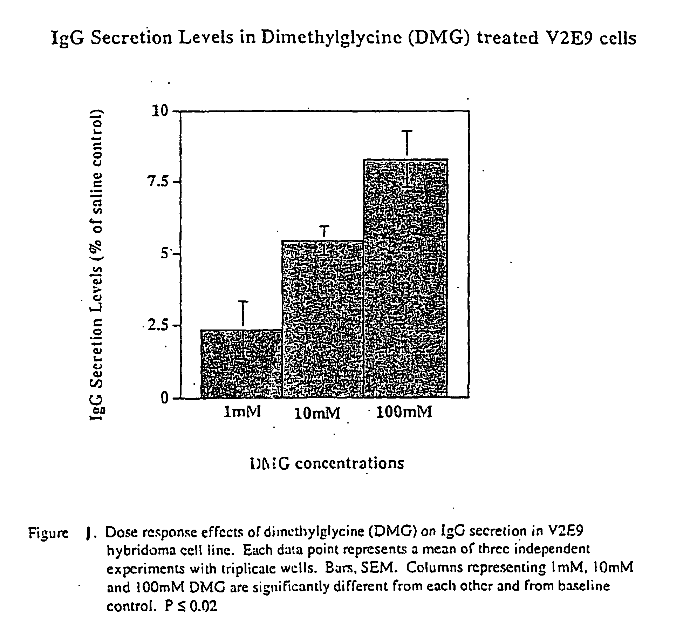

Figure 1 - Dose response effects of IgG Secretion Levels in Dimethylglycine (DMG) treated V2E9 cells
Figure 2 - Dose response effects of Dimethylglycine (DMG) on TNF alpha secretion in stimulated (LPS) THP-1 cell line
Figure 3 - Dose response effects of Dimethylglycine (DMG) on IL-1 secretion in U-937 cell line
Figure 4 - Dose response effects of Dimethylglycine (DMG) on IL-2 secretion in Jurkat E6-1 cell line
Figure 5 - Dose response effects of Dimethylglycine (DMG) on IL-2 secretion in EL-4 cell line
Figure 6 - Dose response effects of Dimethylglycine (DMG) on IL-6 secretion in LS 174 T cell line
Figure 7 - Dose response effects of Dimethylglycine (DMG) on TNF alpha secretion in peripheral blood mononuclear cells
Figure 8 - Dose response effects of Dimethylglycine (DMG) on IL-1 secretion in peripheral blood mononuclear cells
Figure 9 - Dose response effects of Dimethylglycine (DMG) on IL-2 secretion in peripheral blood mononuclear cells
Figure 10 - Inhibitory effect of dimethylglycine (DMG) treated and stimulated (PMA and lonomycin) cytokine producing cell lines on the proliferation of A375.S2 melanoma cells as observed in co-culture.
Figure 11 - Inhibitory effect of dimethylglycine (DMG) treated and stimulated (PMA and lonomycin) cytokine producing cell lines on the proliferation of B16 melanoma cells as observed in co-culture.
Figure 12 - Inhibitory effect of dimethylglycine (DMG) treated stimulated (PMA and lonomycin) Jurkat E6-1 cell line on the proliferation of Hs 294T melanoma cells as observed in co-culture.
Figure 13 - Inhibitory effect of dimethylglycine (DMG) treated peripheral blood mononuclear cells (PBMC) on the proliferation of A375.S2 melanoma cells as observed in co-culture.
Figure 14 - Inhibitory effect of dimethylglycine (DMG) treated peripheral blood mononuclear cells (PBMC) on the proliferation of Hs 294T melanoma cells as observed in co-culture.
Figure 15 - Inhibitory effect cytokine producing cell lines on the proliferation of dimethylglycine (DMG) treated A375.S2 melanoma cells as observed in co-culture.
Figure 16 - Inhibitory effect of naive cytokine producing cell lines on the proliferation of dimethylglycine (DMG) treated B16 melanoma cells as observed in co-culture.
Figure 17 - Inhibitory effect of dimethylglycine (DMG) treated Jurkat E6-1 cell line on the proliferation of MCF-7 breast cancer cells as observed in co-culture.
Figure 18 - Inhibitory effect of dimethylglycine (DMG) treated Jurkat E6-1 cell line on the proliferation of T47D breast cancer cell line as observed in co-culture.
Figure 19 - Inhibitory effect of dimethylglycine (DMG) treated peripheral blood mononuclear cells (PBMC) on the proliferation of MCF-7 breast cancer cell line as observed in co-culture.
Figure 20 - Inhibitory effect of dimethylglycine (DMG) treated peripheral blood mononuclear cells (PBMC) on the proliferation of T47D breast cancer cell line as observed in co-culture.
Figure 21 - Dose dependent effect of DMG on the cytoxicity of B16 mouse melanoma cells.
Figure 22 - Dose dependent effect of DMG on the cytoxicity of Caski human cervical carcinoma cells.
Figure 23 - Dose dependent effect of DMG on the cytoxicity of Caski human cervical carcinoma cells.
Figure 24 - Dose dependent effect of DMG on the cytoxicity of SiHa human cervical carcinoma cells.
Figure 25 - Dose dependent effect of DMG on the cytoxicity of SiHa human cervical carcinoma cells.
Figure 26 - Dose dependent effect of DMG on the cytoxicity of prostate adenocarcenoma cells..

In the foregoing and in the following examples, all temperatures are set forth uncorrected in degrees Celsius and unless otherwise indicated, all parts and percentages are by weight.

### EXAMPLES:

A proliferation assay was designed to determine the ability of DMG treated cytokine producing cells to inhibit growth of melanoma cell lines, breast cancer cell lines, a prostate cancer cell line, and cervical cancer cell lines. The cytokine bioassays utilizing cell proliferation assays were performed to assess modulation of inflammatory cytokine production by DMG. Cytokine bioassays are well known in the art. (Thorpe R: Developments in Biological Standardization 1999, 97:61-71 and Mire-Sluis AR: Journal of Immunological Methods 1995, 187 (2):191-199)

The cell lines and murine B16 as indicator cells were used to observe the modulating effects of DMG. The optimum cell number per well for each cell line was determined after titration assay. Stimulated and unstimulated cytokine producing cell lines (Jurkat e6-1, EL-4, MG-63 and U-937) were treated with DMG for 24 hours prior to culture with cancer cell lines. Human melanoma cell lines A375.S2, HS294T; Mouse melanoma cell line B16.F10; human breast cancer cell lines MCF-7, BT-20, and T47D; human prostrate cell Iline PC-3 and human Cervical cell lines Caski and SiHa were used to determine the effects of DMG.

The preceding examples can be repeated with similar success by substituting the generically or specifically described coatings of this invention for those used in the preceding examples:

### Materials and Methods

### Cell lines and Cell Culture

The cell lines used in this study were V2E9, THP-1, L-929, U-937, A375.S2, jurkat E6-1, EL-4, CTLL-2, HT-2, LS174T, 7TD1, MG-63, B16 and Hs 294T. Characteristics of each cell line including cytokine production and/or sensitivity are presented in Table 1.

**Table I. List of Cytokine producing cell lines and their corresponding Responder cell lines**

| CELL LINES | CYTOKINES PRODUCED | RESPONDER CELL LINES |
|---|---|---|
| THP- 1 | TNF-α | L-929 (sensitive to TNF-α) |
| U-937 | IL-I | A375.S2 (sensitive to IL-1) |
| Jurkat E6-1 | IL-2 | CTLL-2 (proliferates in presence of IL-2) HT-2 (proliferates in presence of IL-2) |
| EL-4 | IL-2 | CTLL-2 (proliferates in presence ot IL-2) HT-2 (proliferates in presence of IL-2) |
| LS 174T | IL-6 | 7TDI (proliferates in presence of IL-6) |

All the cell lines with the exception of V2E9 were obtained from the ATCC. V2E9 is a B cell hybridoma constitutively secreting mouse IgG against the β1 subunit of chicken intergrin. V2E9 cells were grown in Ex-Cell 610 medium (JRH Biosciences) supplemented with 0% or 10% fetal bovine serum (FBS, Gibco BRL) and 1% Antibiotic-Antimycotic (Gibco BRL). All the other cell lines were cultured as per ATCC recommendations with regard to media and respective supplements. CTLL-2 and HT-2 are cytokine dependent cell lines requiring the addition of IL-2, whereas 7TD1 requires the addition of IL-6. All cell lines were grown at 37°C in a humidified 5% CO₂ incubator except EL-4 mouse thymoma cells which required 10% CO₂. V2E9 cells were adapted to grow in serum free media in a gradual manner prior to the hybridoma experiments.

### Isolation of Peripheral Blood Mononuclear Cells (PBMC)

Heparinized peripheral blood from healthy control volunteers was collected and layered onto room temperature (RT) polysucrose sodium diatriazoate (Histopaque 1077, Sigma Chemical Co.) in 15-ml conical tubes. These were centrifuged at 400 X g for 30 minutes. Mononuclear cells were collected and washed in 10-ml phosphate buffered saline (PBS). Following a 10 min centrifugation and 250 X g, the PBS were decanted and the wash step repeated. Cells were counted in a hemocytometer and adjusted to 2X10⁶ cells/ml. These cells were later used in cytokine bioassays, Co-culture and in flow cytometry.

### Preparation of Dimethylglycine (DMG)

DMG was prepared as a 1 M solution by dissolving 1.03g of DMG free base (MW 103.1) in a 10 ml of phosphate buffered saline (PBS) (pH 7.4). This solution was filter sterilized using a 0.22µ Acrodisc filter (Gelman Sciences). Serial dilutions of DMG were made ranging from 1 pM to 1M. Initial studies revealed the activity of DMG to range between 1 mM to 500 mM; hence all cell culture experiments utilized 1 mM to 100 mM of DMG.

### Modulation of Antibody Production in V2E9 Hybridoma Cells

V2E9 cells adapted in serum-free media were harvested, enumerated and passed into 24 well plates in Ex-cell 610 media at a concentration of 5X10⁶ cells/ml. One hundred µl of increasing concentrations of DMG was added to the wells. Nine hundred µl of Ex-cell media was added to bring the total volume in each well to 2000 µl. Treated V2E9 cells and controls were incubated for 48 hours at 37°C. Supernatants were drawn off from the wells and assayed for IgG levels using ELISA.

### Enzyme Linked Immunoassay for IgG Quantitation in V2E9 Hybridomas

Goat affinity purified antibody to mouse IgG (Cappel, Organaon Teknika Corp.) was diluted in PBS (pH 7.4) to a concentration of 0.1 µg/µl. Ninety-six well ELISA plates (Coming) were coated with 100 µl of this buffered suspension for an hour at 37°C. After an hour the plates were air-dried for an additional hour at room temperature (RT). Then the plates were washed twice with PBS. Next, the plates were blocked with freshly prepared Blotto (Skim milk in PBS) for 60 minutes at 37°C. Plates were then rinsed again twice with PBS and dried. One hundred µl of the supernatants from the treatment and control wells were added to the plates and allowed to incubate for an hour at 37°C. Following incubation, plates were rinsed twice with Blotto and PBS. One hundred µl of peroxidase conjugate goat affinity purified antibody to mouse IgG (Cappel, Organaon Teknika Corp.) diluted 1:100 in PBS were added to each well. Plates were incubated for 30 minutes at 37°C, and then rinsed four times with Blotto, and twice with PBS. Fresh color developer [24.3 ml of 0.1 M Citric acid (Sigma), 25.7 ml of 0.2 M Na₂HPO₄ (Fisher Scientific), 50 ml distilled water, 40 mg o-phenylenediamine (Sigma) and 40 µl of 30% H₂O₂ (Fisher Scientific)] were added to each well (100 µl/well) and allowed to react for 20 minutes in the dark at RT. Reactions were terminated by the addition of 50 µl of 2.5 M H₂SO₄ to each well. The plates were read using a BIO-RAD benchmark microplate reader at 490 nm.

### Modulation of Cytokine Production in Cell Lines (Cytokine Bioassays)

Cytokine bioassays utilizing cell proliferation assays were performed to assess modulation of inflammatory cytokine production by DMG (House- Development in biological Standardization 1999 (97) 13-19). Supernatants from cytokine producing cell lines (untreated control and DMG treated), were added to responder cell lines specific for that cytokine. Proliferation or inhibition of these responder/indicator cells signal the extent of cytokine secreted by the producer cell lines. The optimum cell number/well for each cell line was determined after a titration assay. All suspension producer cell lines such at THP-1, Jurkat E6-1, EL-4 and U-937 were plated on 48 well plates in a concentration of 5X10⁶ cells/well, where as adherent producer cells such as LS174T were plated at a concentration of 5X10⁵ cells/well. Suspension responder cell lines (CTLL-2 and 7TD-1) were used at a concentration of 1X10⁶ cellstwell in a 96 well plate, whereas the adherent responder cell lines (L-929 and A375.S2) were plated at a concentration of 3X10⁴ cells/well and 4X10⁴ cells/well respectively. THP-1 cells, which secrete Tumor Necrosis Factor-alpha (TNF-α), when stimulated with lipopolysaccharide (LPS), were treated with increasing concentrations of DMG for a period of 24 hours at 37°C in a humidified 5% CO₂ incubator. Supernatants from these cells were then added to the indicator cell line (I929), which is sensitive to TNF-α. L-929 cells were then incubated for 24 hours at 37°C in a humidified 5% CO₂ incubator. After incubation, MTS-PMS solution, (Cell Titer 96 Aqueous Kit, Promega, Madison, WI) was added to each well as recommended by the manufacturer (31). Detection of proliferation or inhibition is based on a colorimetric assay system utilizing the novel tetrazolium reagent, MTS, which is reduced to a water soluble formazan dye, via the alternate electron acceptor PMS (phenazine methosulphate) in the mitochondria of living cells. Samples are read at 4 hours using a BIO-RAD benchmark microplate reader at 490 nm. The amount of color produced is directly proportional to the number of viable cells. The experiments were carried out in triplicate and repeated three to six times for each cytokine. Similarly, U-937 cells, which secrete Interieukin-1 (IL-1) constitutively, were treated with DMG and the supernatants added to the sensitive cell line A375.S2. Likewise, Jurkat E6-1 and EL-4 mouse thymoma cells which when stimulated with lonomycin and Phorbol Myristate Acetate (PMA) secrete IL-2, were treated with DMG and the supernatants were added to the responder cell line CTLL-2. Finally, LS174T colon adenocarcinoma cells that constitutively secrete IL-6 were treated with DMG and the supernatants added to the responder 7TD1-cell line.

### Modulation of Cytokine Production in Peripheral Blood Mononuclear Cells (PBMC)

Modulation of inflammatory cytokine production in Peripheral Blood Mononuclear Cells (PBMC) by DMG, was determined by the use of cytokine bioassays Supernatants of DMG treated and untreated PBMC which produce cytokines (producers) on stimulation with mitogens (PMA and/or lonomycin), were added to responder cell lines specific for the respective cytokine. Proliferation or inhibition of these indicator cells signal the extent of cytokine secretion by the producer cell lines. The optimum cell numberavell for each cell line was determined after a titration assay. PBMC were plated on 48 well plates in a concentration of 5X10⁶ cellslwell. Responder cell line, CTLL-2 used in the detection of IL-2 was added to a 96 well plate at a concentration of 1X10⁶ cells/well. The adherent responder cell lines, L-929, used to detect TNF-α and A375.S2, used to detect IL-1, were plated at a concentration of 3X10⁴ cells/well and 4X10⁴ respectively. PBMC were stimulated with PMA and lonomycin for the production of TNF-α, IL-1 and IL-2 and treated with increasing concentrations of DMG for a period of 24 hours at 37°C in a humidified 5% CO₂ incubator. Supernatants from these cells were then added to the indicator cell lines, L929: sensitive to TNF-α, CTLL-2: dependent on IL-2 for its proliferation, and A375 S2: sensitive to IL-1 respectively . These indicator cell lines were then incubated for 24 hours at 37°C in a humidified 5% CO₂ incubator. After incubation, MTS-PMS solution, (Cell Titer 96 Aqueous Kit, Promega, Madison, WI) was added to each well as recommended by the manufacturer (31). Detection of proliferation or inhibition is based on a colorimetric assay system utilizing the novel tetrazolium reagent, MTS, which is reduced to.a water soluble formazan dye, via the altemate electron acceptor PMS (phenazine methosulphate) in the mitochondria of living cells. Samples are read at 4 hours using a BIO-RAD benchmark microplate reader at 490 nm. The amount of color produced is directly proportional to the number of viable cells. The experiments were carried out in triplicate and repeated three to six times for each cytokine.

### Modulation of Breast, Cervical and Prostrate Cell Proliferation in Co-culture with DMG treated Cytokine Producing Cell lines and with DMG treated PBMC

A proliferation assay was designed to determine the ability of DMG treated cytokine producing cells to inhibit growth of human breast cancer cell lines MCF-7 , BT-20 and T47D, human prostrate cancer PC-3 cell line, human cervical cancer Caski and SiHa cell lines and melanoma cell lines. Three melanoma cell lines, human A375.S2 and Hs 294T, and murine B16 as indicator cells were used to observe the modulating effects of DMG. The optimum cell number/well for each cell line was determined after a titration assay. Stimulated and unstimulated cytokine producing cell lines (Jurkat E6-1, EL-4, MG-63 and U-937) were treated with DMG for 24 hr prior to culture with melanoma cells. Similarly, PBMC were also treated with DMG and cultured with melanoma cells. To evaluate the exact nature of DMG in mediated melanoma inhibition, melanoma cells (B16 and A375.S2) were preteated with DMG for a period of 24 hours prior to culture with cytokine producing cells.

### Modulation of inflammatory cytokine production in cell lines

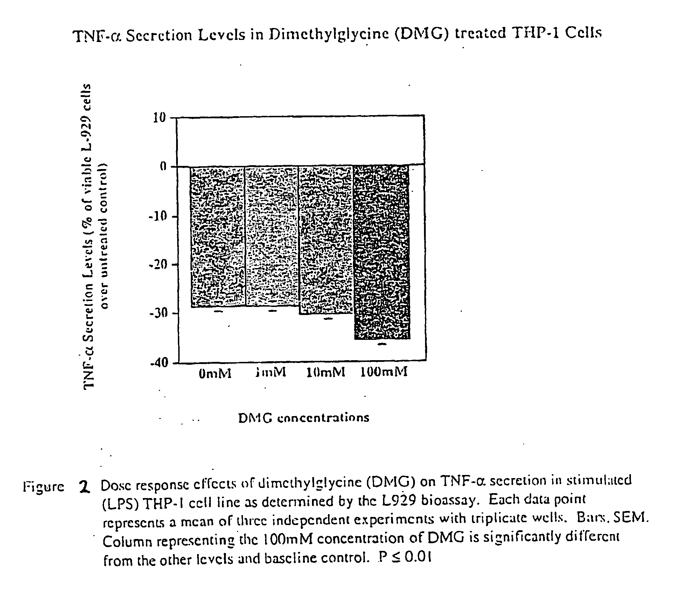

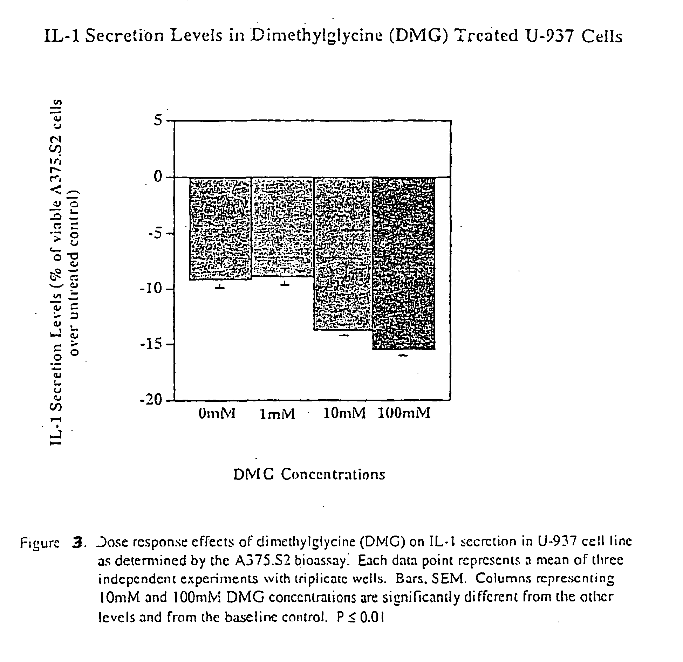

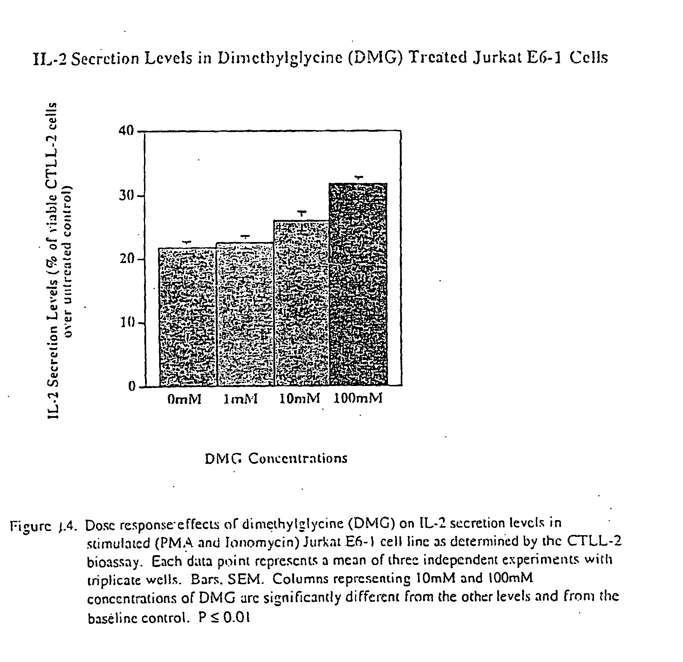

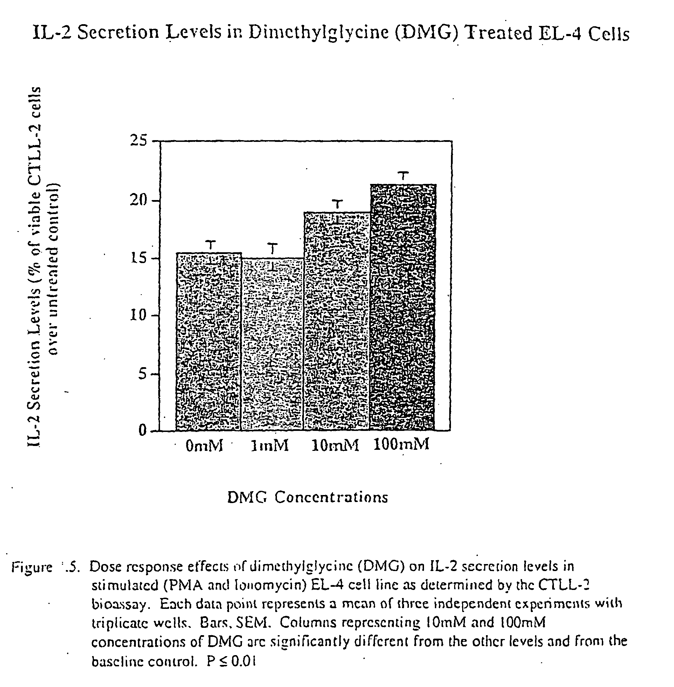

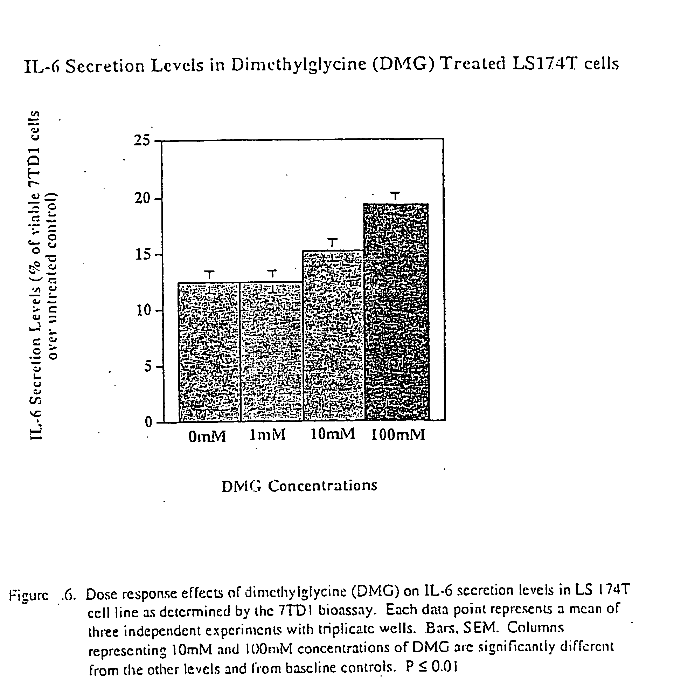

DMG was then tested for its ability to modulate cytokine expression in monocytic and lymphocytic cell lines. Cytokine bioassay results (Fig 2-6) demonstrated that DMG significantly increased inflammatory cytokine levels in a dose dependent manner. One hundred mM DMG significantly increased TNF-α levels by 7% over baseline control levels as measured by the proliferation of L-929 sensitive (Fig 2). Similarly, in U-937 cells that secrete IL-1 constitutively, 10 mM and 100 mM DMG treatments increased IL-1 secretion levels. This increase in IL-1 is detected by a decrease in the proliferation of sensitive A375.S2 cells (Fig 3). DMG treatment with 10 mM and 100 mM led to significant increase of 4.5% and 6.5% respectively. Comparable results were also obtained in Jurkat E6-1 and EL-4 cells that secrete IL-2. Supernatants of DMG treated Jurkat E6-1 and EL-4 cell suspensions induced increased proliferation rates in CTLL-2 cell line, implying augmented secretion levels of IL-2. Significant augmentations in IL-2 levels in the Jurkat E6-1 and EL-4 supernatants were observed with 100 mM DMG treatment leading to increases in proliferation of CTLL-2 by 10% and 6%, while 10 mM DMG treatment increased IL-2 secretion in these two cell lines by 3% and 2.5% (Fig 4 and 5). Likewise IL-6 production levels in LS 174T were increased significantly by 7% in presence of 100 mM DMG (Fig 6).

### Modulation of Cytokine expression in Peripheral Blood Mononuclear Cells (PBMC)

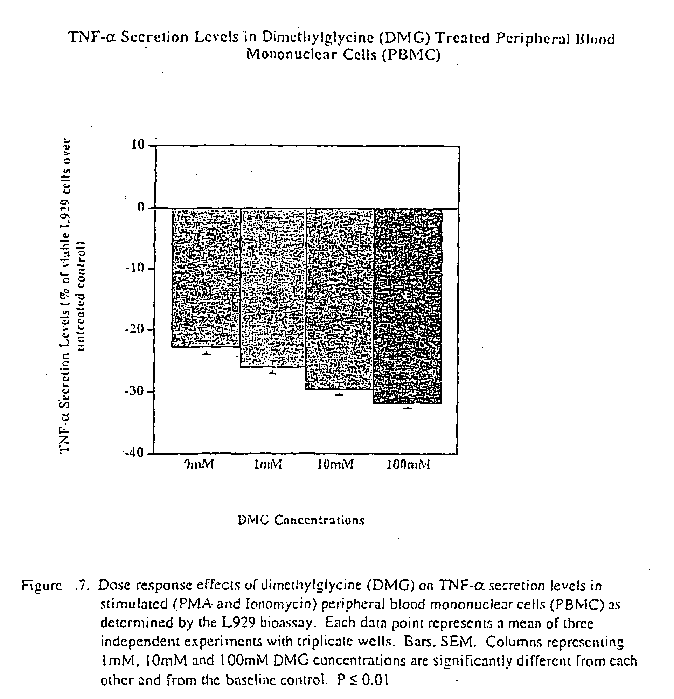

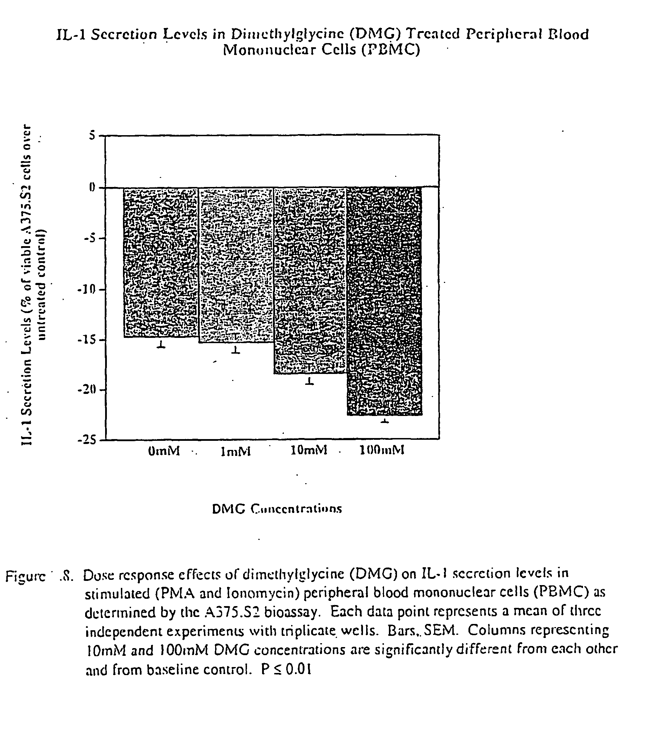

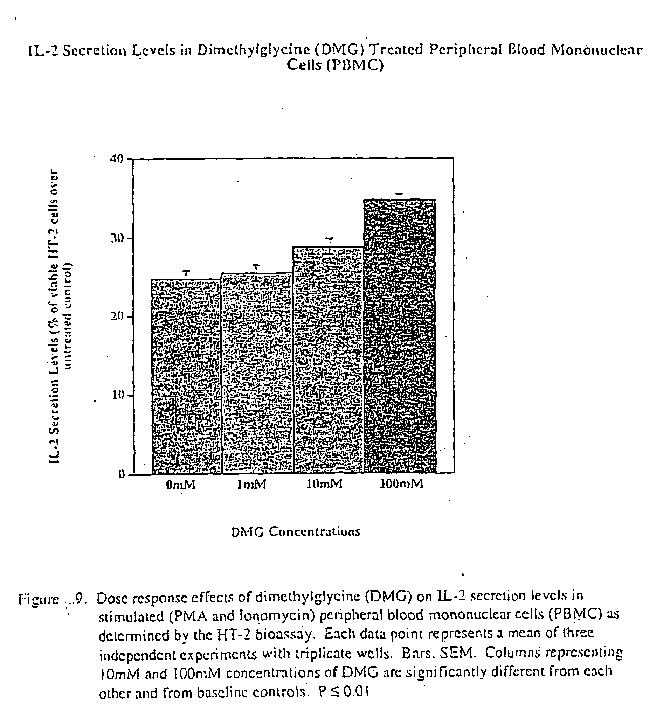

PMBC were stimulated with PMA and lonomycin and treated with varying doses of DMG to assay for the levels of inflammatory cytokines. Cytokine bioassay results (Fig 7-9) demonstrate the effect of DMG in increasing inflammatory cytokine secretion in a dose dependent manner. At 100 mM concentration of DMG, TNF-α were significantly raised by 9% over stimulated baseline controls, as measured by the increased proliferation of the indicator L-929 cells (Fig 7). Similarly IL-1 and IL-2 expression levels were significantly enhanced in PBMC in the presence of 100 mM DMG by 8% and 10% respectively as observed by the growth rate of A375.S2 and HT-2 responder cell lines.

### Modulation of Tumor Cell Proliferation by DMG

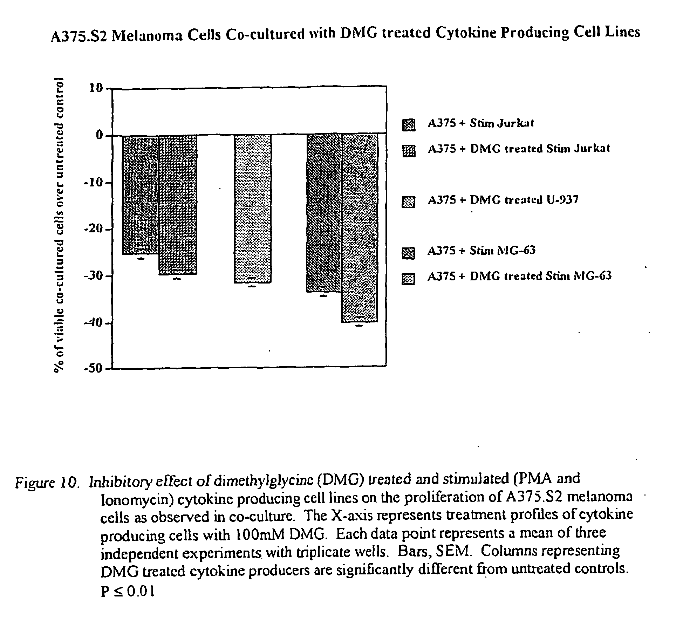

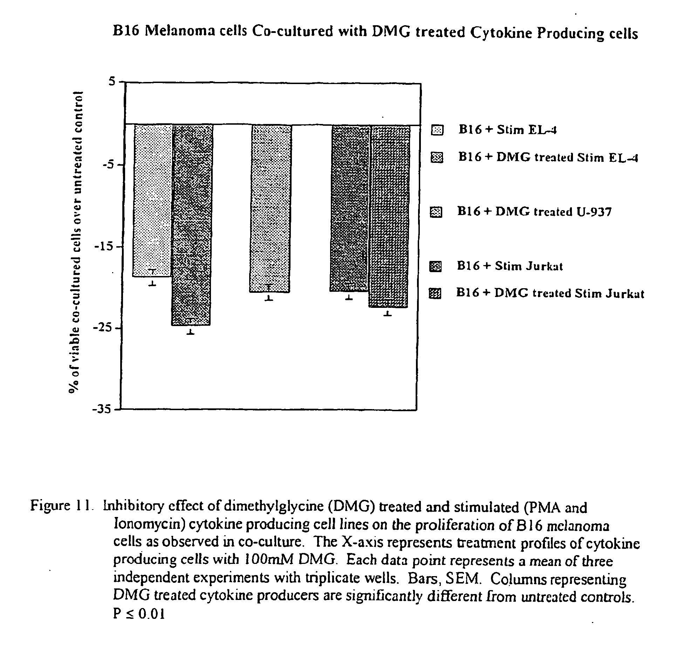

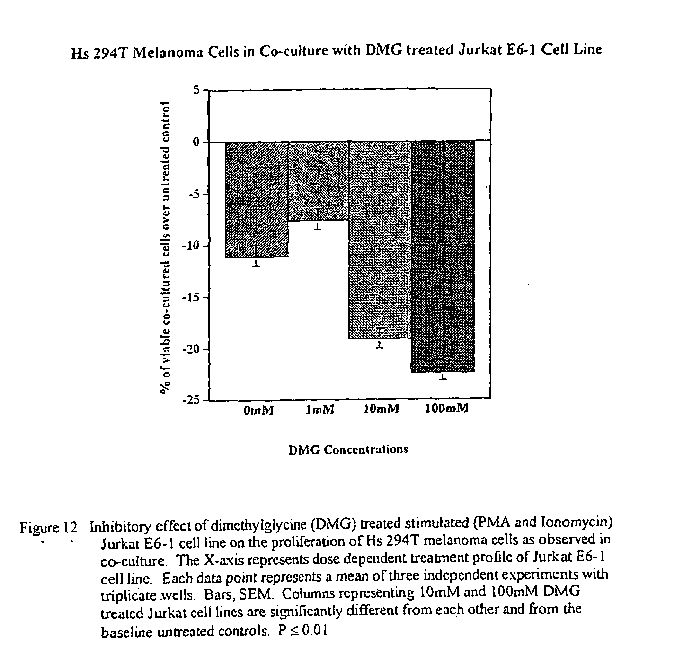

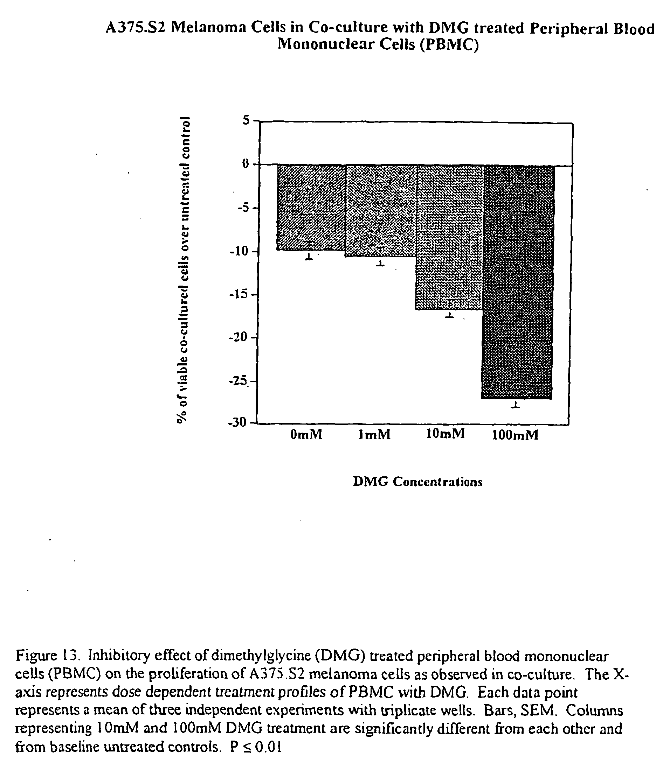

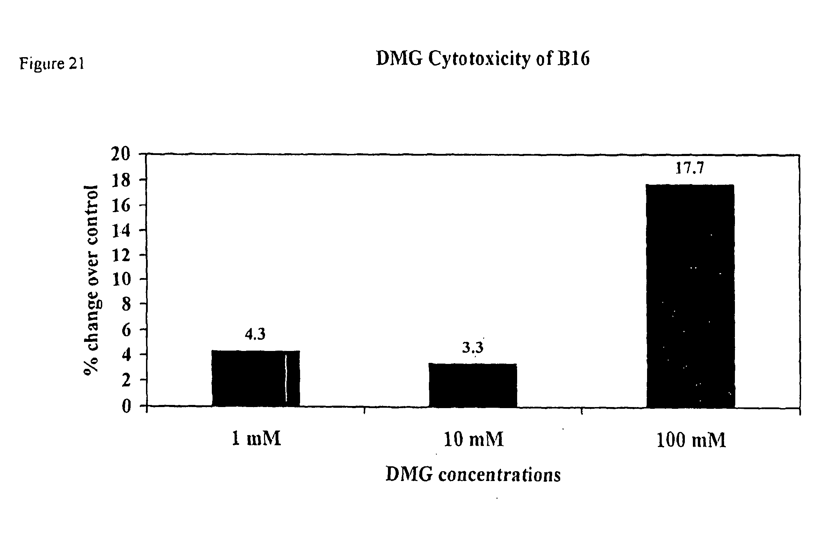

Since DMG modulated inflammatory cytokine production in cell lines and in PBMC (Fig 2-9), the compound was tested for its ability to indirectly affect the growth of melanoma cell lines. In this study cytokine producing cells (Jurkat E6-1, EL-4, U-937 and MG-63 were stimulated with lonomycin and Phorbol Myristate Acetate (PMA), treated with DMG and co-cultured with melanoma cell lines (A375.S2, Hs 294T and B16). Fig 10-21 demonstrated the effect of these cell lines in inhibiting melanoma cell lines. DMG enhanced IL-2 secretion in Jurkat E6-1 which led to increased inhibition of melanoma cells by 8% over baseline stimulated control. DMG treated U-937 expressed enhanced levels of IL-1, which was indicated by a 30% decrease in the proliferation of sensitive A375.S2 cells. MG-63, which produced IFN-β, when stimulated also displayed increased level of melanoma inhibition. Similar inhibitory effects were observed in B16 and Hs 294T melanoma cell lines, which were co-cultured with DMG-treated Jurkat E6-1, EL-4 and U-937 cell lines.

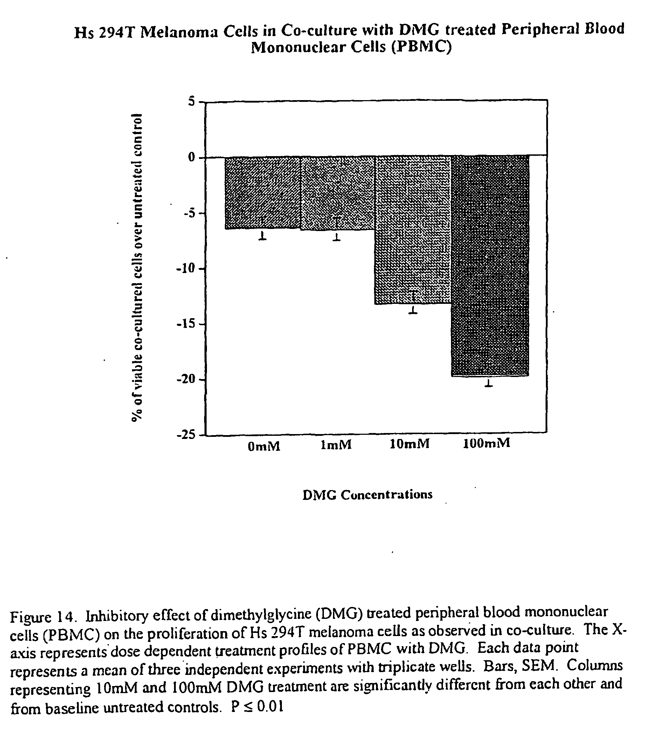

To detect whether this cytokine dependent melanoma inhibition was also produced by PBMC, these cells were stimulated with lonomycin and PMA, and then treated with increasing concentrations of DMG followed by co-culture with A375.S2 and Hs 294T melanoma cells . Significant dose dependent reductions in A375.S2 proliferation were detected with DMG treated PBMC. PBMC treated with 100 mM DMG led to a significant proliferation decreases in melanoma cell growth by 17% over stimulated baseline control. Fig 14 depicts the dose dependent effect of DMG in augmenting melanoma inhibition by PBMC, wherein significant inhibitory effect of 13% over controls was observed at the 100 mM DMG level.

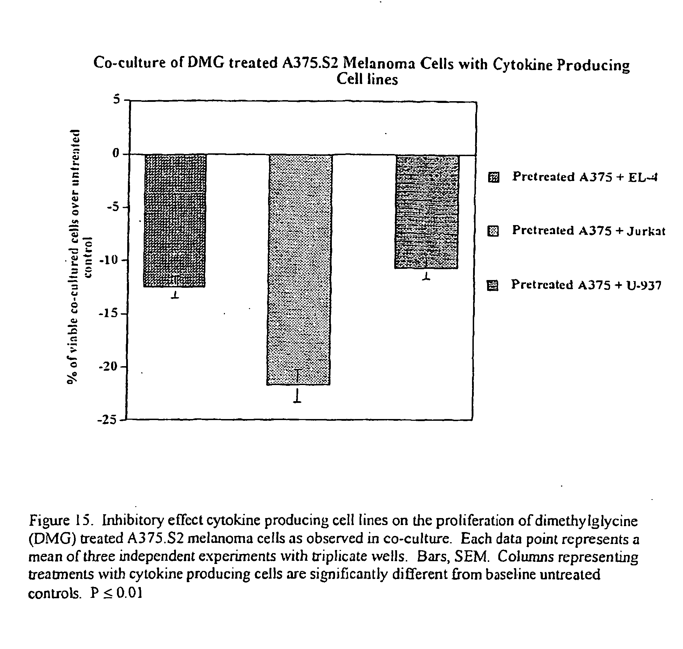

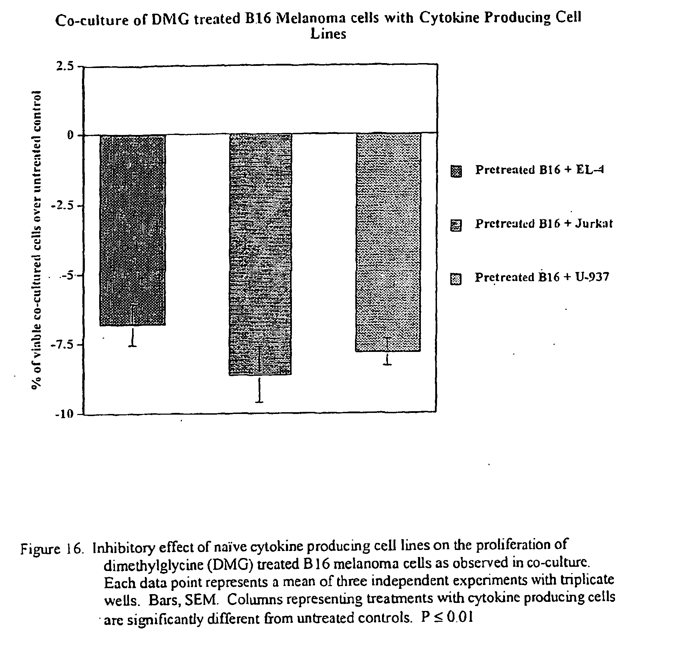

Studies involving pretreatment of the melanoma cell lines with DMG were conducted to understand the nature of melanoma growth inhibition induced by cytokine producing cells. This reduction in growth rate could primarily be a product of increased inflammatory cytokine production or a dual effect dependent on cell-cell contact and expression of surface factors as well as cytokines. The ability of cytokine producing cells in facilitating melanoma inhibition even in the absence of direct DMG treatment is indicated in Fig 15-16. This ability to kill melanoma cells is indicative of cell-cell contact and/or expression of surface factors. Species specificity in melanoma growth inhibition was observed in Fig 16. EL-4 mouse thymoma cells which secretes IL-2 significantly inhibited B16 mouse melanoma cells than human Jurkat E6-1 that also produces IL-2.

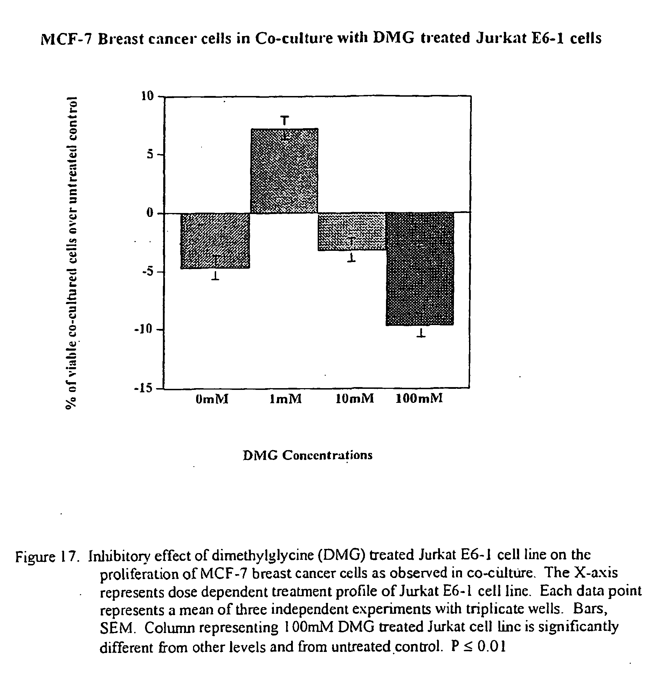

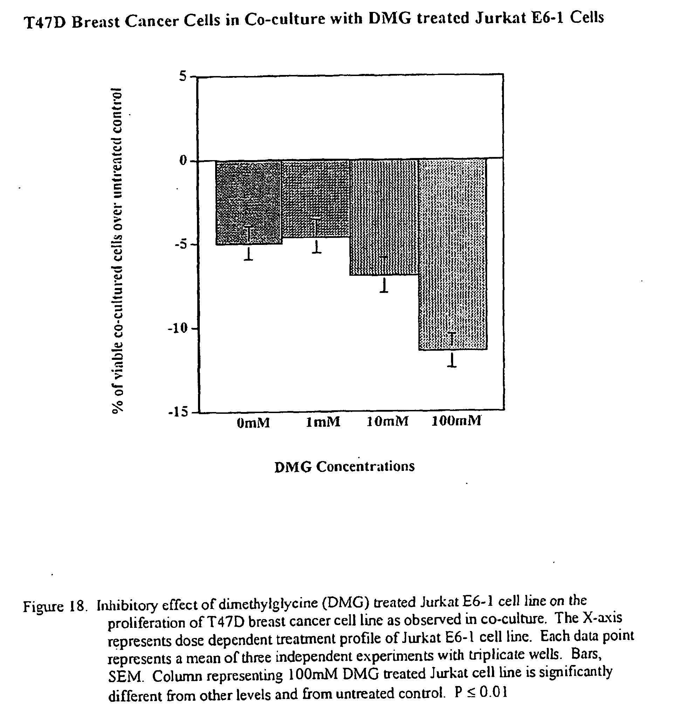

Human breast cancer cell lines MCF-7 , BT-20 and T47D, human prostrate cancer PC-3 cell line, human cervical cancer Caski and SiHa cell lines and three melanoma cell lines, human A375.S2 and Hs 294T, and murine B16 were used in co-culture experiments. DMG treated human Jurkat E6-1 cell line demonstrated similar significant ability in inhibiting breast cancer cells as with melanoma cells (Fig 17 and 18).

### EXAMPLE I

Jurkat E6-1 cell line was treated with DMG for 24 hours prior to culture.with T47F human breast ductal adenocarcinoma cell line. Inhibition of T47D cells followed a typical dose dependent effect (fig 18).

### EXAMPLE II

Jurkat E6-1 cell line was treated with DMG for 24 hours prior to culture with MCF-7 mammary carcinoma cell line. Inhibition of MCF-7 cells followed a dose dependent effect (fig 17).

### EXAMPLE III

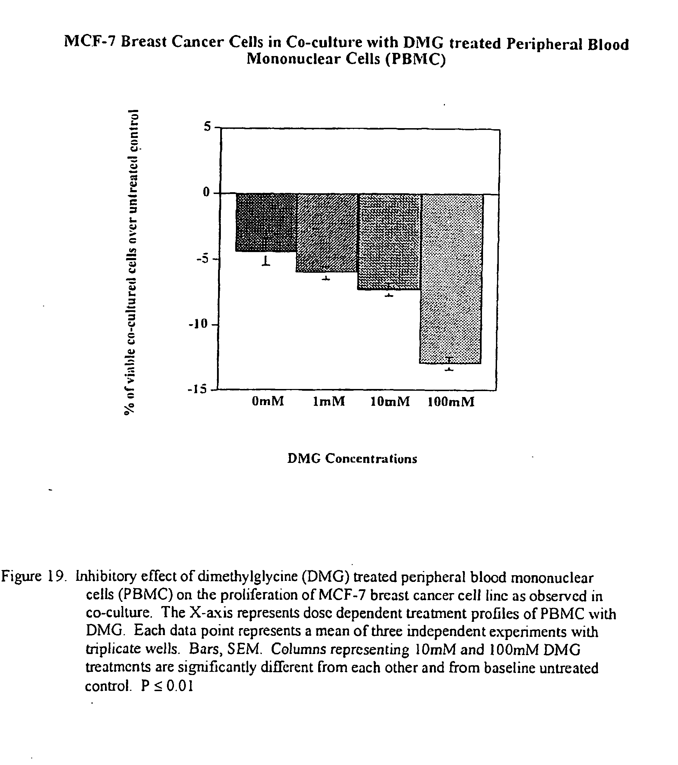

DMG treated stimulated PBMC were co-cultured with MCF-7 cell lines. DMG treated PBMC demonstrated a dose response inhibition of cell lines. (Fig 19 )

### EXAMPLE IV

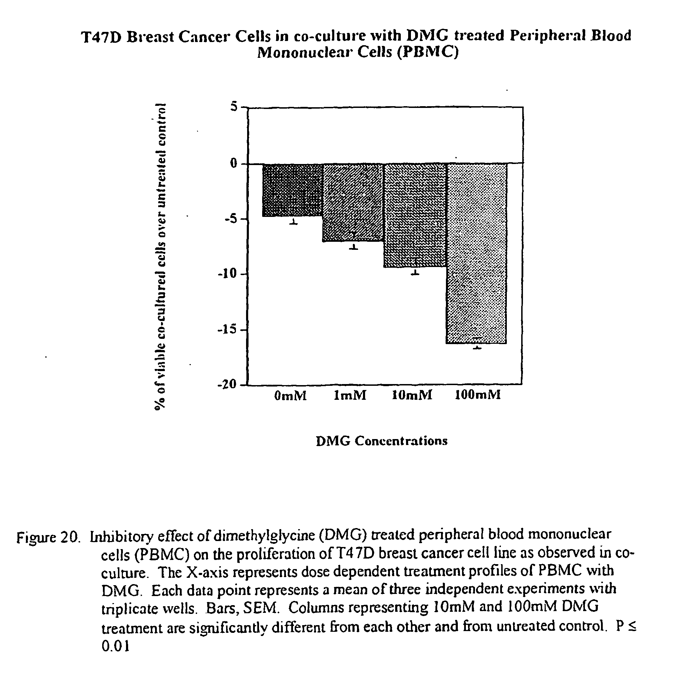

DMG treated stimulated PBMC were co-cultured with T47D cell lines . DMG treated PBMC demonstrated a dose response inhibition of cell lines. (Fig 20)

### EXAMPLE V

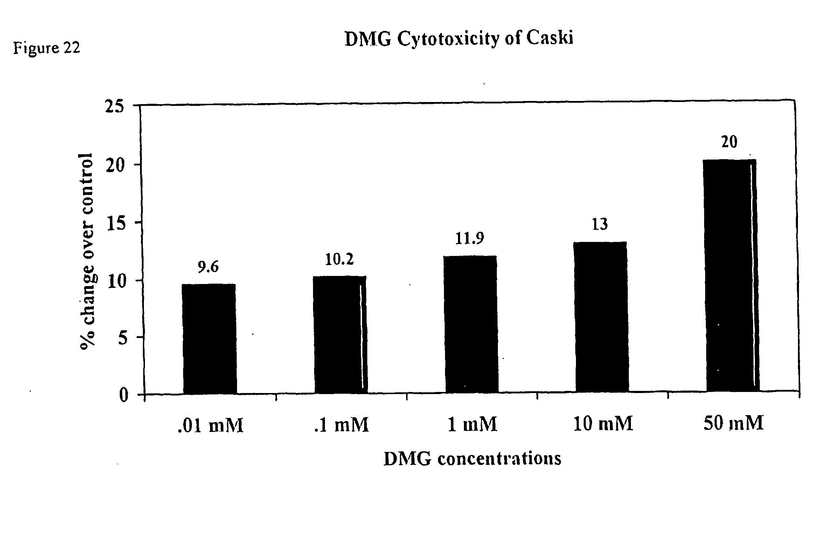

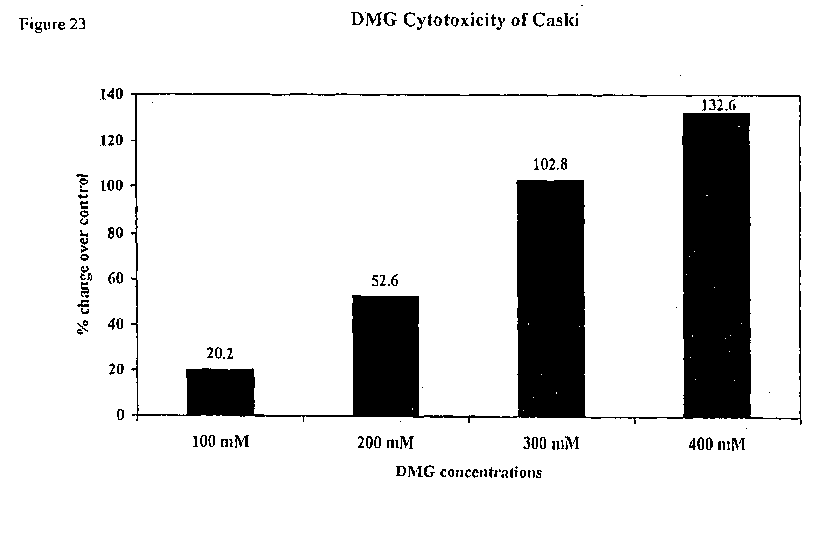

Jurkat E6-1 cell line was treated with DMG for 24 hours prior to culture with Caski, a human cervical cell line . DMG treated human Jurkat E6-1 cell line demonstrated a dose response inhibition of cell lines. (Figure 22 - 23)

### EXAMPLE VI

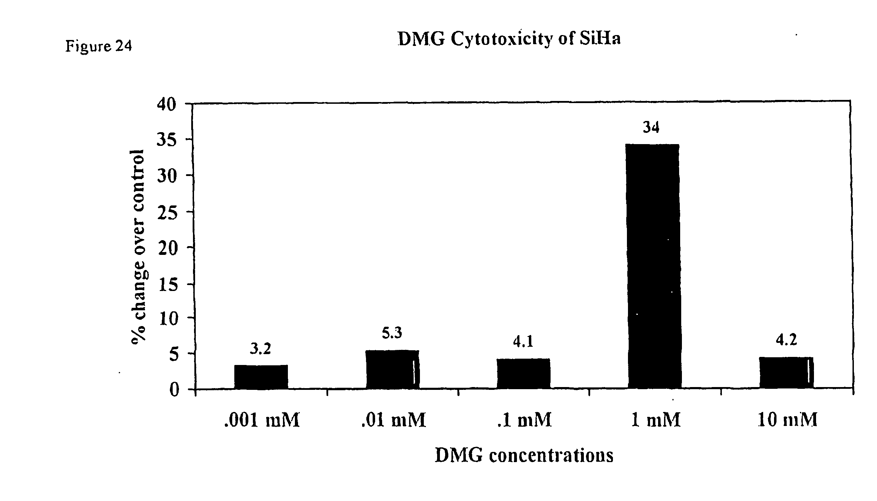

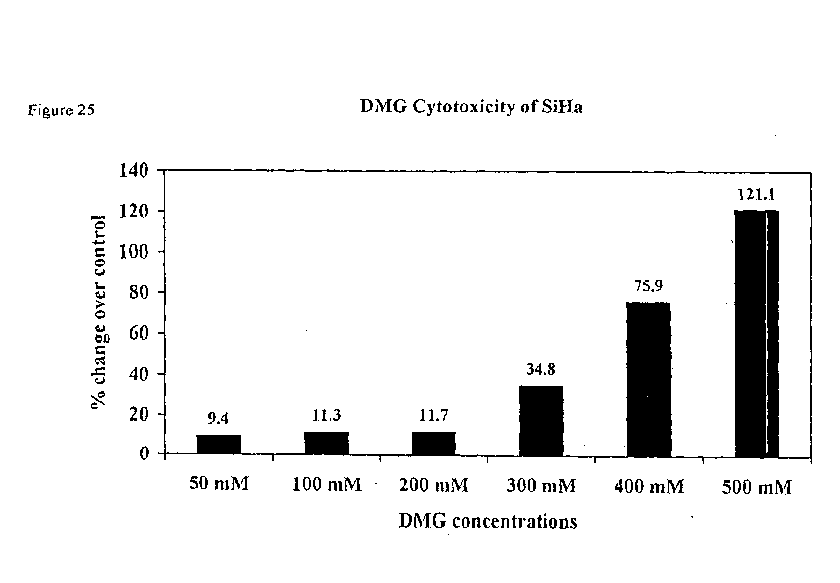

Jurkat E6-1 cell line was treated with DMG for 24 hours prior to culture with SiHa, a human cervical cell line . DMG treated human Jurkat E6-1 cell line demonstrated a dose response inhibition of cell lines. (Figure 24-25)

### EXAMPLE VII

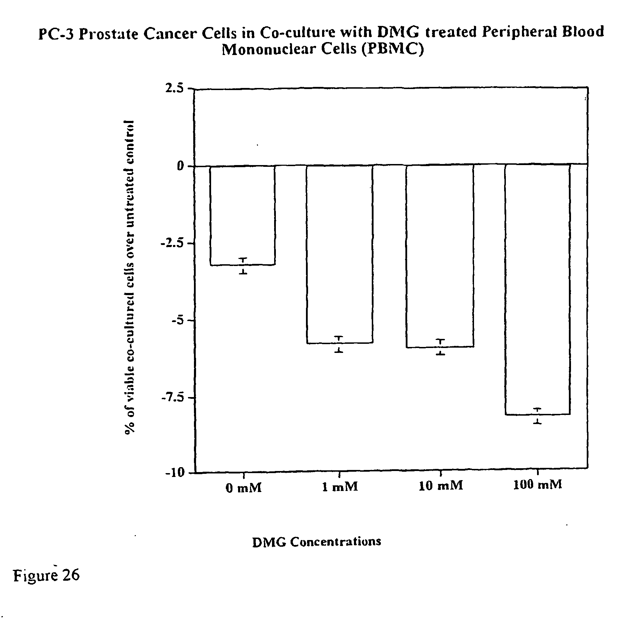

DMG treated stimulated PBMC were co-cultured with prostate adenocarcinoma cell line PC-3. DMG treated PBMC demonstrated a dose response inhibition of cell lines. (Figure 26)

## Claims

1. Use of N,N-dimethylglycine or a pharmaceutically acceptable salt thereof for the manufacture of a medicament for inhibiting the metastasis of cervical, breast or prostate cancer in a patient.

2. Use according to claim 1 for systemic or oral administration.

3. Use according to claim 1 for administration in an amount of 1-500 mg/kg/day, preferably in an amount of 20-200 mg/kg/day.

4. Use of N,N-dimethylglycine or a pharmaceutically acceptable salt thereof for the manufacture of a medicament for inhibiting the formation of cervical, breast or prostate cancer cells in a patient or for decreasing the risk of cervical, breast or prostate cancer in a patient who is at risk for cervical, breast or prostate cancer.

5. Use according to claim 4 for administration to a patient who does not have cancer.

6. Use according to claim 1 or 4 for administration in admixture with a pharmaceutically acceptable carrier.

7. Use according to claim 4 for systemic or oral administration.

8. Use according to claim 4 for administration of the N,N-dimethylglycine in an amount of 1-500 mg/kg/day, preferably in an amount of 10-100 mg/kg/day.

9. Use of N,N-dimethylglycine or a pharmaceutically acceptable salt thereof for the manufacture of a medicament for treating cervical, breast or prostate cancer in a patient.

10. Use according to claim 9 for systemic or oral administration.

11. Use according to claim 9 for administration in an amount of 1-500 mg/kg/day, preferably in an amount of 20-200 mg/kg/day

## Patentansprüche

1. Verwendung von N,N-Dimethylglycin oder eines pharmazeutisch annehmbaren Salzes desselben für die Herstellung eines Medikaments zur Hemmung von Metastasen von Gebärmutterhals-, Brust- oder Prostatakrebs bei einem Patienten.

2. Verwendung nach Anspruch 1 für die systemische oder orale Verabreichung.

3. Verwendung nach Anspruch 1 für die Verabreichung in einer Menge von 1 bis 500 mg/kg/Tag, vorzugsweise in einer Menge von 20 bis 200 mg/kg/Tag.

4. Verwendung von N,N-Dimethylglycin oder eines pharmazeutisch annehmbaren Salzes desselben für die. Herstellung eines Medikaments zur Hemmung der Bildung von Gebärmutterhals-, Brust- oder Prostatakrebszellen bei einem Patienten oder zur Verringerung der Gefahr von Gebärmutterhals-, Brust- oder Prostatakrebs bei einem Patienten, bei dem ein Risiko für Gebärmutterhals-, Brust- oder Prostatakrebs besteht.

5. Verwendung nach Anspruch 4 für die Verabreichung an einen Patienten, bei dem kein Krebs vorliegt.

6. Verwendung nach Anspruch 1 oder 4 für die Verabreichung im Gemisch mit einem pharmazeutisch annehmbaren Träger.

7. Verwendung nach Anspruch 4 für die systemische oder orale Verabreichung.

8. Verwendung nach Anspruch 4 für die Verabreichung von N,N-Dimethylglycin in einer Menge von 1 bis 500 mg/kg/Tag, vorzugsweise in einer Menge von 10 bis 100 mg/kg/Tag.

9. Verwendung von N,N-Dimethylglycin oder eines pharmazeutisch annehmbaren Salzes desselben für die Herstellung eines Medikaments zur Behandlung von Gebärmutterhals-, Brust- oder Prostatakrebs bei einem Patienten.

10. Verwendung nach Anspruch 9 für die systemische oder orale Verabreichung.

11. Verwendung nach Anspruch 9 für die Verabreichung in einer Menge von 1 bis 500 mg/kg/Tag, vorzugsweise in einer Menge von 20 bis 200 mg/kg/Tag.

## Revendications

1. Utilisation de N,N-diméthylglycine ou d'un sel de celle-ci pharmaceutiquement admissible pour préparer un médicament destiné à inhiber la métastase du cancer du col de l'utérus, du sein ou de la prostate chez un patient.

2. Utilisation selon la revendication 1 pour une administration systémique ou orale.

3. Utilisation selon la revendication 1 en administrant à une quantité de 1 à 500 mg/kg/jour, de préférence en une quantité de 20 à 200 mg/kg/jour.

4. Utilisation de N,N-diméthylglycine ou d'un sel de celle-ci pharmaceutiquement admissible pour préparer un médicament destiné à inhiber la formation de cellules cancéreuses du col de l'utérus, du sein ou de la prostate chez un patient ou pour diminuer le risque de cancer du col de l'utérus, du sein ou de la prostate chez un patient qui est un sujet à risque pour le cancer du col de l'utérus, du sein ou de la prostate.

5. Utilisation selon la revendication 4 pour une administration à un patient qui ne présente pas de cancer.

6. Utilisation selon la revendication 1 ou 4 pour une administration en mélange avec un vecteur pharmaceutiquement admissible.

7. Utilisation selon la revendication 4 pour une administration systémique ou orale.

8. Utilisation selon la revendication 4 pour l'administration de N,N-diméthyl-glycine en une quantité de 1 à 500 mg/kg/jour, de préférence en une quantité de 10 à 100 mg/kg/jour.

9. Utilisation de N,N-diméthylglycine ou d'un sel de celle-ci pharmaceutiquement admissible pour préparer un médicament destiné au traitement du cancer du col de l'utérus, du sein ou de la prostate chez un patient.

10. Utilisation selon la revendication 9 pour une administration systémique ou orale.

11. Utilisation selon la revendication 9 pour l'administration en une quantité de 1 à 500 mg/kg/jour, de préférence en une quantité de 20 à 200 mg/kg/jour.
